# EUROPEAN PATENT APPLICATION

(11) **EP 4 461 974 A1**
(43) Date of publication of application: **13.11.2024**
(21) Application number: 22918320.7
(22) Date of filing: 02.12.2022
(51) Int. Cl.: F16B 21/07, F16B 5/00, A61B 90/00, A61B 34/30, A61B 50/30

(54) **CLAMPING MECHANISM, INSTRUMENT BOX AND STERILE ISOLATION PLATE ASSEMBLY**

(30) Priority: 06.01.2022 CN 202210008887
(71) Applicant: Agibot Medtech (Suzhou) Co., Ltd., Suzhou, Jiangsu 215123 (CN)
(72) Inventor: WANG, Yupu, Suzhou, Jiangsu 215123 (CN); PENG, Cheng, Suzhou, Jiangsu 215123 (CN); XU, Min, Suzhou, Jiangsu 215123 (CN)
(74) Representative: Pfenning, Meinig & Partner mbB
(86) International application number: PCT/CN2022/136086
(87) International publication number: WO 2023/130868

(57) **Abstract**

The present disclosure is related to a clamping mechanism, an instrument cassette, and a sterile isolation plate assembly. The clamping mechanism includes a base plate and a pair of clamping assemblies. Each clamping assembly is fixedly connected to an upper surface of the base plate, and has an engaging/clamping position and a disengaging/unlocking position. Each clamping assembly includes a moving member, at least two clamping members, and several elastic members respectively abutting against respective clamping members. The moving member extends in a front-rear direction and is configured to be movable back and forth between the engaging/clamping position and the disengaging/unlocking position. The clamping member includes an upper and a lower end portion. The upper end portion is fixedly connected to or abuts against the corresponding moving member, and the lower end portion is of a hook-shaped structure. The clamping assembly is movable from the engaging/clamping position to the disengaging/unlocking position under an action of an external force and compress the corresponding elastic member, and movable from the disengaging/unlocking position to the engaging/clamping position under an action of the elastic member. The instrument cassette and the sterile isolation plate can be stably connected by means of the clamping mechanism, thereby avoiding shaking and even detachment.

## Description

This application claims the priority to Chinese Patent Application No. 202210008887.3, filed with the China National Intellectual Property Administration on January 06, 2022 and entitled "CLAMPING MECHANISM, INSTRUMENT CASSETTE, AND STERILE ISOLATION PLATE ASSEMBLY", the entirety thereof are incorporated herein by reference.

### FIELD OF THE INVENTION

The present disclosure relates to the field of medical devices, and in particular, to a clamping mechanism, an instrument cassette, and a sterile isolation plate assembly.

### BACKGROUND OF THE INVENTION

Surgical robots are becoming more sophisticated and widely used in minimally invasive surgical operations. During operation of a surgical robot, to avoid contamination of an instrument cassette caused by a power box or replacement of the instrument cassette, a sterile isolation plate needs to be arranged between the power box and the instrument cassette to isolate the power box from the instrument cassette, thereby ensuring a sterile environment for a surgical area and reducing risks of infection.

Currently, a clamping mechanism is usually used to achieve a detachable connection between the isolation plate and the instrument cassette. However, if the clamping is not stable, shaking or even detachment may occur between the isolation plate and the corresponding instrument cassette. Shaking is then transmitted via a transmission rod of the surgical robot to an effector terminal, which may affect surgical accuracy slightest or more seriously may cause irreversible damage to a patient.

### SUMMARY OF THE INVENTION

For a related technical problem of an unstable clamping/engagement and even detachment of a sterile isolation plate from an instrument cassette, an objective of the present disclosure is to provide a clamping mechanism, an instrument cassette, and a sterile isolation plate assembly for a stable clamping/engagement.

To achieve the foregoing objective, a first aspect of the present disclosure provides a clamping mechanism, including a base plate and a pair of clamping assemblies that are disposed opposite to each other on left and right sides, wherein both the clamping assemblies are fixedly connected to a same surface of the base plate; each clamping assembly includes a moving member, at least two clamping members, and several elastic members in one-to-one correspondence with the clamping members; wherein each clamping member has an engaging/clamping position and a disengaging/unlocking position; the clamping member is configured to be able to clamp a third unit when in the engaging/clamping position and allow the third unit to detach when in the disengaging/unlocking position; wherein each clamping assembly is so configured that: the moving member can move along the surface of the base plate under an action of an external force, drive the at least two clamping members to move from the engaging/clamping position to the disengaging/unlocking position, and compress the several elastic members to enable the at least two clamping members to move from the disengaging/unlocking position to the engaging/clamping position under an action of an elastic restoring force.

In the foregoing technical solution, preferably, each moving member extends in a front-rear direction; the base plate is provided with two sets of guide rails that are movably connected to a pair of the moving members, respectively; the two sets of guide rails are both configured to extend along a left-right direction; and the moving member can move back and forth along the corresponding set of guide rails. Further preferably, the moving member includes a pressing portion extending along an up-down direction and a guiding portion extending along the left-right direction; and each guiding portion is movably connected to the corresponding set of guide rails.

In the foregoing technical solutions, preferably, several clamping seats in one-to-one correspondence with the clamping members are fixedly disposed on the base plate; and each clamping member has a rotating shaft and is in a shaft/hole connection to the corresponding clamping seat through the rotating shaft. Further preferably, each clamping member includes an upper end portion and a lower end portion that are respectively disposed on two sides of the rotating shaft; each upper end portion is fixedly connected to or abuts against the corresponding moving member; each lower end portion is configured to be of a hook-shaped structure; and when moving under an action of an external force, the moving member drives the corresponding upper end portion to rotate, so that the lower end portion of the clamping member switches between the disengaging/unlocking position and the engaging/clamping position. Further preferably, the base plate is provided with several through slots in one-to-one correspondence with the clamping members; each clamping member passes through the corresponding through slot and exposes its lower end portion; and the elastic member abuts against the upper end portion of the corresponding clamping member. The clamping member switches between the disengaging/unlocking position and the engaging/clamping position.

In the foregoing preferable solutions, further preferably, the elastic member is a spring; several elastic bases, of which a quantity is consistent with that of the elastic members, that respectively abut against the elastic members are also arranged on the base plate; and each elastic member applies a force to the corresponding clamping member to maintain the clamping member at the engaging/clamping position.

In the foregoing preferable solutions, further preferably, the elastic member is an elastic leaf of an inverted U-shaped structure; the elastic member and the corresponding clamping member are constructed as an integral part; several elastic bases in one-to-one correspondence with the elastic members are also fixedly disposed on the base plate; and each elastic member abuts against the corresponding elastic base and applies a force to the corresponding clamping member to maintain the clamping member at the engaging/clamping position.

In the foregoing preferable solutions, further preferably, the clamping member is provided with a hollow portion; the elastic member is a torsion spring and is arranged at a position of the corresponding hollow portion; each elastic member abuts against the corresponding clamping member and the base plate at two end portions ; and each elastic member applies a force to the corresponding clamping member to maintain the clamping member at the engaging/clamping position.

According to a second aspect, the present disclosure provides an instrument cassette, where the instrument cassette includes: a mounting base; a pair of first clamping assemblies that are arranged relative to each other on left and right sides on the mounting base, where each first clamping assembly includes a first moving member extending along a front-rear direction, at least two first clamping members that abut against or are fixedly connected to the first moving members, and several first elastic members with a quantity consistent with that of the first clamping members; the first clamping member is elastically connected to the mounting base by using the corresponding first elastic member, and has a first engaging/clamping position and a first disengaging/unlocking position; and each first moving member is provided with a first pressing portion to which an external force can be applied; an instrument upper housing, where the instrument upper housing is of a box-shaped structure with an open lower portion, and is provided with a pair of pressing slots that can allow a pair of the pressing portions to be exposed, respectively; and an instrument transmission device, disposed on the mounting base and located on an inner side of the instrument upper housing. Each first moving member is configured to be able to drive, under an action of an external force, the corresponding first clamping member to move from the first engaging/clamping position to the first disengaging/unlocking position, and compress the corresponding first elastic member to enable the first clamping member to move from the first disengaging/unlocking position to the first engaging/clamping position under an action of an elastic restoring force. The first clamping member is configured to be able to clamp a third unit when in the first engaging/clamping position and allow the third unit to detach when in the first disengaging/unlocking position.

In the foregoing technical solution, preferably, each first clamping assembly is fixedly connected to an upper surface of the mounting base; the mounting base is provided with first through slots in one-to-one correspondence with the first clamping members; and each first clamping member passes through the corresponding first through slot and exposes a lower end portion.

According to a third aspect, the present disclosure provides a sterile isolation plate assembly, including: a second base plate; a pair of second clamping assemblies that are arranged relative to each other on left and right sides on the second base plate, where each second clamping assembly includes a second moving member extending along a front-rear direction, at least two second clamping members that abut against or are fixedly connected to the second moving members, and several second elastic members respectively abutting against the second clamping members; each second clamping member has a second engaging/clamping position and a second disengaging/unlocking position; and each second moving member is provided with a second pressing portion to which an external force can be applied; and a sterile isolation plate, fixedly connected to or detachably connected to the second base plate, where the sterile isolation plate is provided with a pair of pressing slots that can allow a pair of the second pressing portions to be exposed, respectively; and a pair of second clamping assemblies are both located on an inner side of the sterile isolation plate. The second moving member is configured to be able to drive, under an action of an external force, the corresponding second clamping member to move from the second engaging/clamping position to the second disengaging/unlocking position, and enable the second clamping member to move from the second disengaging/unlocking position to the second engaging/clamping position under an action of an elastic restoring force. The several second clamping members can clamp a third unit when in the second engaging/clamping position and allow the third unit to detach when in the second disengaging/unlocking position.

In the foregoing technical solution, preferably, the second base plate is provided with several second through slots in one-to-one correspondence with the second clamping members; and the second clamping member passes through the corresponding second through slot and exposes a lower end portion.

In the foregoing technical solution, preferably, the third unit is an instrument cassette; when the sterile isolation plate assembly is clamped with the instrument cassette, the sterile isolation plate is located on a lower side of the instrument cassette; and a pair of second clamping assemblies are clamped with a mounting base of the instrument cassette.

In the foregoing technical solution, preferably, the third unit is a power box; when the sterile isolation plate assembly is clamped with the power box, the sterile isolation plate is located on an upper side of the power box; and a pair of second clamping assemblies are clamped with a top cover of the power box.

Compared with prior art, the clamping mechanism of the present disclosure is provided with two sets of clamping assemblies that are disposed on left and right sides, and each set of clamping assemblies is equipped with at least two clamping members. At least four clamping points are provided and meanwhile convenient operation is ensured (it is only required to press one pair of clamping assemblies in left and right directions during unlocking); by this, sufficient stability may be ensured during an engagement/clamping of the instrument cassette or the power box, thereby avoiding shaking or even detachment of the instrument cassette or the power box.

### BRIEF DESCRIPTION OF THE DRAWINGS

FIG. 1 is a schematic perspective view of a clamping mechanism according to a first implementation of the present disclosure;
FIG. 2 is an exploded diagram of a clamping mechanism shown in FIG. 1;
FIG. 3 is a schematic perspective view of a clamping member of a clamping mechanism shown in FIG. 1;
FIG. 4 is a semi-sectional front view of a clamping mechanism shown in FIG. 1, where the clamping mechanism is engaged/clamped with a third unit, and a clamping member is at an engaging/clamping position;
FIG. 5 is another semi-sectional front view of a clamping mechanism shown in FIG. 1, where the clamping mechanism is disengaged/detached from a third unit, and a clamping member is at a disengaging/unlocking position;
FIG. 6 is a schematic perspective view of a clamping mechanism according to a second implementation of the present disclosure;
FIG. 7 is a schematic perspective view of an elastic member of a clamping member of a clamping mechanism shown in FIG. 6;
FIG. 8 is a schematic perspective view of a clamping mechanism according to a third implementation of the present disclosure;
FIG. 9 is a schematic perspective view of an elastic member of a clamping member of a clamping mechanism shown in FIG. 8;
FIG. 10 is a schematic perspective view of an instrument cassette according to the present disclosure, where the instrument cassette is engaged with a sterile isolation plate;
FIG. 11 schematically illustrates a disassembly of an instrument cassette and a sterile isolation plate shown in FIG. 10;
FIG. 12 schematically illustrates an application scenario of an instrument cassette shown in FIG. 10, where the instrument cassette is mounted with an effector;
FIG. 13 is a schematic perspective view of a sterile isolation plate assembly according to the present disclosure;
FIG. 14 is an exploded diagram of a sterile isolation plate assembly shown in FIG. 13;
FIG. 15 schematically illustrates an application scenario of a sterile isolation plate assembly shown in FIG. 13, where the sterile isolation plate assembly is illustrated in an exploded way;
FIG. 16 is a semi-sectional front view of an application scenario shown in FIG. 15, where a second clamping member is at its engaging/clamping position; and
FIG. 17 schematically illustrates an application scenario of an instrument cassette and a sterile isolation plate assembly according to the present disclosure.

### DETAILED DESCRIPTION OF THE EMBODIMENTS

Technical content, structural features, achieved objectives, and effects of the present disclosure will be described in detail. Technical solutions in embodiments of this application will be described below in combination with accompanying drawings in the embodiments of this application. Obviously, the described embodiments are merely some embodiments of this application and are not all embodiments. In the following description, for a purpose of explanation, a lot of specific details are elaborated to provide detailed description to various exemplary embodiments or implementations of the present disclosure. However, the various exemplary embodiments may also be implemented without these specific details or in one or more equivalent arrangements. In addition, the various exemplary embodiments may be different, but need not to be exclusive. For example, without departing from the concept of the present disclosure, specific shapes, structures, and characteristics of the exemplary embodiment may be used or implemented in another exemplary embodiment.

In addition, in this application, spatial relative terminologies such as "under", "below", "underneath", "down", "above", "on", "over", "higher", "side" (for example, in a "side wall") are used to describe a relationship between one element and another (other) element as shown in the accompanying drawings. The spatial relative terminologies are intended to include different orientations of a device in use, operation, and/or manufacturing, in addition to orientations described in the accompanying drawings. For example, if the device in the accompanying drawings is turned over, a component described as "below" or "under" another component or feature may be positioned as "above" the other component or feature. Therefore, the exemplary term "below" may include both orientations of over and below. In addition, the device may be positioned in other manners (for example, may be rotated 90 degrees or in other orientations). In this connection, the spatial relative terminologies used herein should be explained accordingly.

In this application, the term "third unit" refers to a component or device that is independent of a clamping mechanism and can be clamped/engaged with or unlocked/disengaged from the clamping mechanism, including but not limited to a sterile isolation plate, an instrument cassette, and a power box involved in this application.

In this application, unless otherwise specified and defined, the term "connect" should be interpretated in a broad way. For example, "connection" may mean a fixed connection, a detachable connection, or to form as an integral; also, "connection" may be a direct connection, or an indirect connection via an intermediate medium.

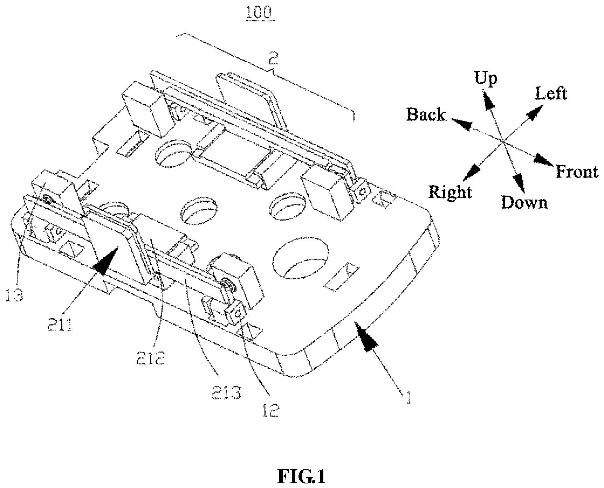

FIG. 1 illustrates a first embodiment provided by the present disclosure - a clamping mechanism 100. The clamping mechanism 100 includes a base plate 1 and a pair of clamping assemblies 2 that are disposed opposite to each other on left and right sides. The pair of clamping assemblies 2 are fixedly connected to an upper surface of the base plate 1.

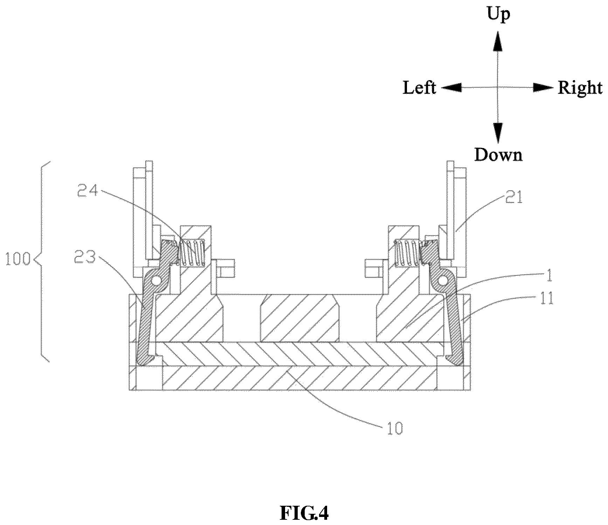

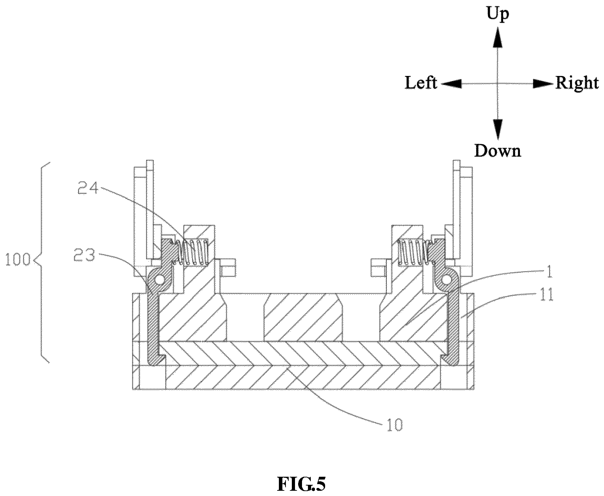

The clamping assembly 2 includes a moving member 21 that can move back and forth along a left-right direction, a guide rail 22 extending along the left-right direction, a pair of clamping members 23 that are respectively provided at a front region and a rear region of the clamping assembly 2, and a pair of elastic members 24 that respectively abut against the pair of clamping members 23. The guide rail 22 is fixedly connected on an upper surface of the base plate 1. The moving member 21 includes a pressing portion 211, a guiding portion 212, and a crossbar portion 213 that are all integrally connected. The pressing portion 211 extends along an up-down direction, and an outer wall of the pressing portion 211 partially extends outwardly to form a force-bearing wall 214, to which an external force may be applied. The guiding portion 212 extends along the left-right direction and is movably coupled to the guide rail 22. The crossbar portion 213 extends along the front-rear direction and abuts against the pair of clamping members 23, simultaneously. Each clamping member 23 has an engaging/clamping position (see FIG. 4) that allows an engagement/clamping with a third unit and a disengaging/unlocking position (see FIG. 5) that allows a detachment of the third unit, and is configured to be movable back and forth between the engaging/clamping position and the disengaging/unlocking position.

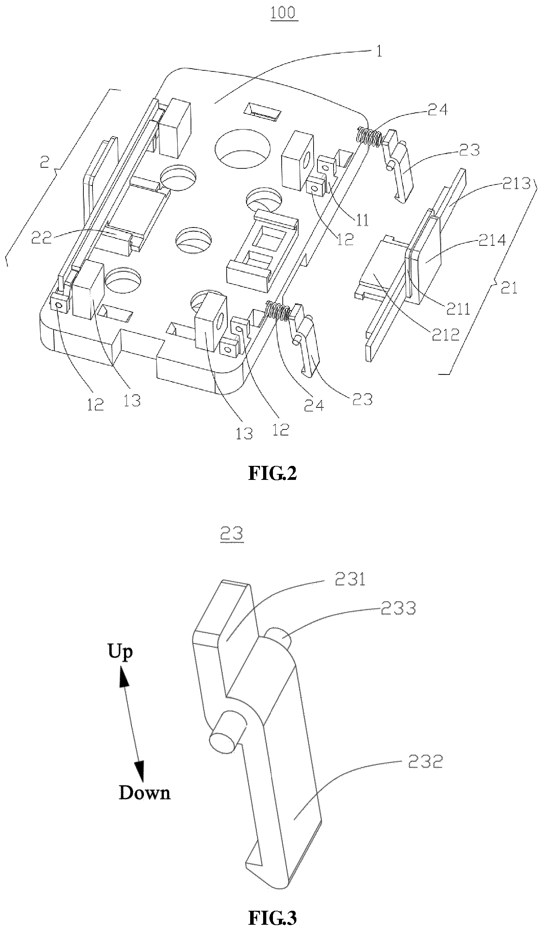

In combination with FIG. 2 and FIG. 3, the base plate 1 has several through slots 11, several pairs of clamping seats 12, and several elastic bases 13, the number of which are the same with the number of clamping members 23. The through slots 11 are provided in the base plate 1, and two clamping seat in each pair of clamping seats 12 are respectively disposed on a front side and rear side of the corresponding through slot 11. The clamping member 23 has an upper end portion 231, a lower end portion 232, and a rotating shaft 233 provided between the upper end portion 231 and the lower end portion 232. The clamping member 23 passes through the corresponding through slot 11, and forms a rotatable shaft/hole connection with the corresponding clamping seats 12 via the rotating shaft 233. The lower end portion 232 of the clamping member 23 is configured to be of a hook-shaped structure to clamp a corresponding third unit. The elastic base 13 is located on an inner side of the corresponding clamping member 23. The elastic member 24 abuts against the elastic base 13 and the upper end portion 231 of the clamping member 23 at two end portions, and provides an outward force to the clamping member 23. The clamping member 23 abuts against the crossbar portion 213 via its upper end portion under an action of the elastic member 24. In this embodiment, the elastic member 24 is a spring, and the elastic base 13 is integrally connected to the base plate 1.

A working principle of the clamping mechanism 100 will be described below in combination with FIG. 4 and FIG. 5. When an external force facing towards an inner side of the clamping mechanism 100 is applied to the force-bearing wall 214, the moving member 21 will move towards the inner side and compresses the pair of elastic members 24. During this process, the lower end portion 232 of the clamping member 23 will move towards the outer side until reaching the disengaging/unlocking position and detaching from the third unit 10. After the external force is removed, the moving member 21 will move towards the outer side under the action of the pair of elastic members 24. The clamping assembly 2 will move from the disengaging/unlocking position to the engaging/clamping position and get engaged/clamped with the third unit 10.

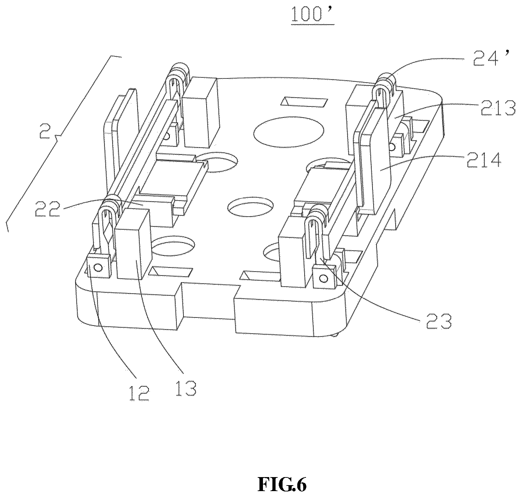

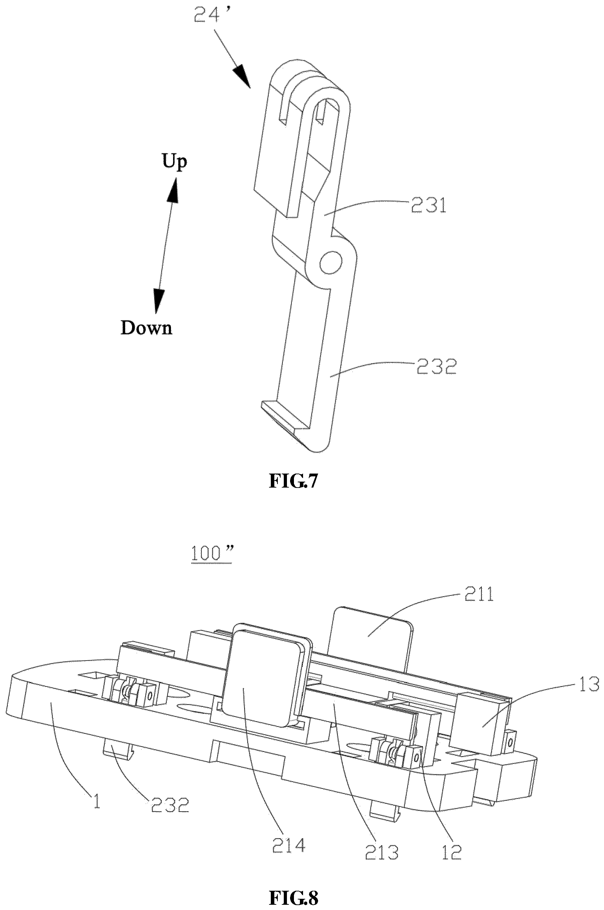

FIG. 6 and FIG. 7 illustrate a second embodiment provided by the present disclosure - a clamping mechanism 100'. A difference between the clamping mechanism 100' and the clamping mechanism 100 is that: the elastic member 24' for the clamping mechanism 100' is an elastic leaf of an inverted U-shaped structure. Each elastic member 24' and the corresponding clamping member 23 are formed as an integral component, and a side surface of each elastic member 24' that is away from the corresponding clamping member 23 abuts against the corresponding elastic base 13. The elastic member 24' generates corresponding deformation under pressure, so as to provide a force to the clamping member 23 to hold it at the engaging/clamping position.

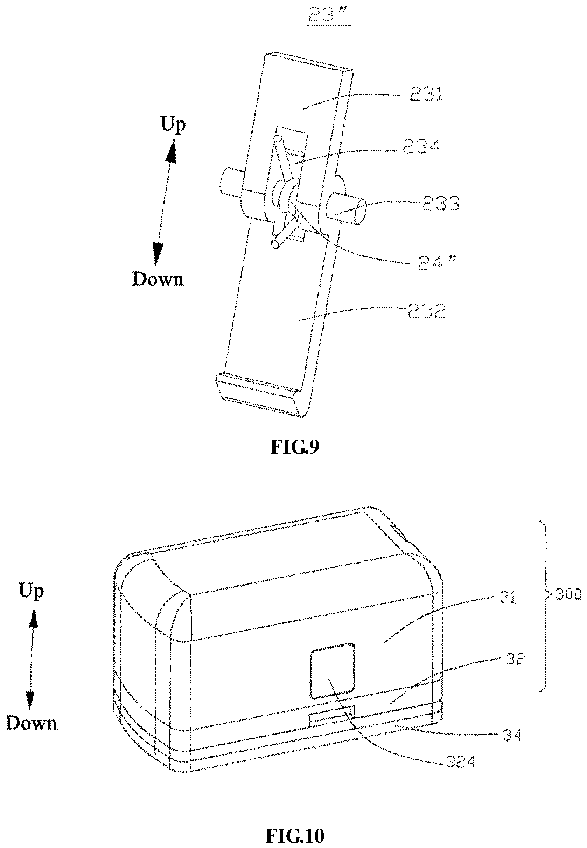

FIG. 8 and FIG. 9 illustrate a third embodiment provided by the present disclosure - a clamping mechanism 100". A difference between the clamping mechanism 100" and the clamping mechanism 100 is that the elastic member 24" for the clamping mechanism 100" is a torsion spring and the clamping member 23" thereof is provided with a hollow portion 234. The elastic member 24" is provided within the hollow portion and abuts against the base plate 1 and the clamping member 23" at two end portions, so as to provide a force urging the clamping member 23" into the engaging/clamping position. In this embodiment, a function of the elastic base 13 is to limit a movement range of the corresponding clamping member 23".

FIG. 10 illustrate a fourth embodiment provided by the present disclosure - an instrument cassette 300. The instrument cassette 300 can be engaged/clamped with a corresponding sterile isolation plate 34, and includes a first clamping mechanism 32, an instrument upper housing 31 located on an upper side of the first clamping mechanism 32, and an instrument transmission device provided within the instrument upper housing 31 (not shown in the figure). The first clamping mechanism 32 may be the clamping mechanism 100, the clamping mechanism 100', or the clamping mechanism 100" provided in the present disclosure. Components of the first clamping mechanism 32 are not elaborated herein.

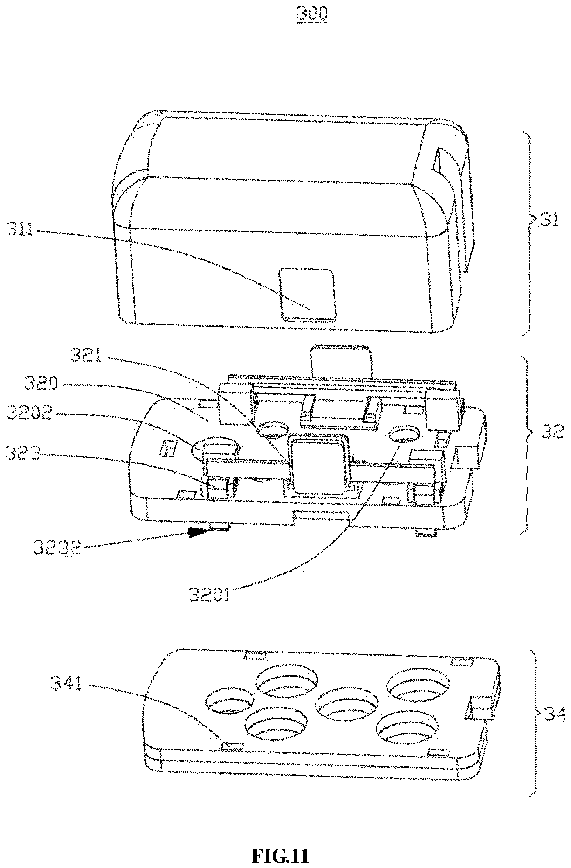

Referring to FIG. 11, the instrument upper housing 31 is provided with a pair of pressing slots 311 that is respectively adapted to a pair of first pressing portions 321. A first force-bearing wall 324 on the first pressing portion 321 extends towards the pressing slot 311 and is flushed with an outer wall surface of the instrument upper housing 31. The instrument upper housing 31 is of a box-shaped structure with a lower opening, and is fixedly connected to an upper surface of a mounting base 320. The instrument upper housing 31 and the mounting base 320 jointly define an instrument chamber 312. The instrument transmission device is arranged in the instrument chamber 312 and is provided on the mounting base 320. The mounting base 320 is also provided with a first power hole 3201 and an effector hole 3202. The instrument transmission device is in a transmission connection to an external power device (such as a power box 43 below) through the first power hole 3201, and is in a transmission connection to a corresponding effector 33 (see the following description) through the first effector hole 3202.

The sterile isolation plate 34 is provided with first clamping slots 341 that are respectively adapted to various first clamping members 323. There is an inner slot structure adapted to a lower end portion 3232 of the corresponding first clamping member 323 on an inner side of the first clamping slot 341 (which is not shown in FIG. 11). The first clamping member 323 is configured to be engaged with the inner slot structure when located at the first engaging/clamping position, and to be separated from the inner slot structure when located at the first disengaging/unlocking position. In this way, the sterile isolation plate 34 may get engaged/clamped and disengaged/unlocked.

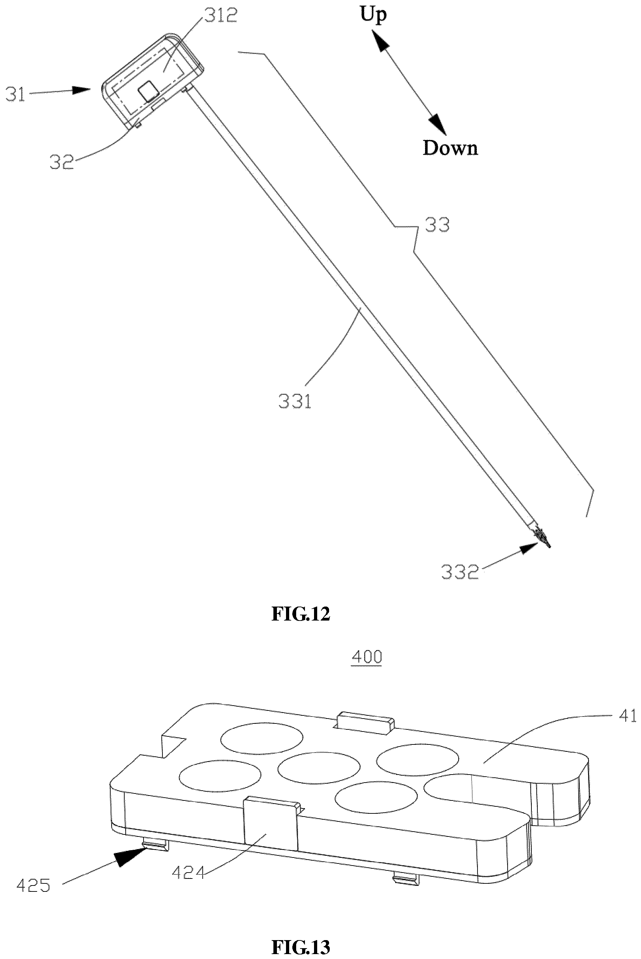

FIG. 12 shows an application scenario of an instrument cassette 300. The instrument cassette 300 is mounted with an effector 33 for operation. The effector 33 includes an effector terminal 332 controlled by the instrument transmission device and an effector shaft 331 extending between the instrument transmission device and the effector terminal 332. The effector terminal 332 is formed by any type of surgical instrument (such as a clamp, a gripper, scissors, anastomoses, a cauterizer, a linear cutter, or a needle holder). The instrument transmission device can control the effector terminal 332 to act for surgical operation. The effector shaft 331 is configured to implement a transmission connection between a control terminal and the effector terminal 332.

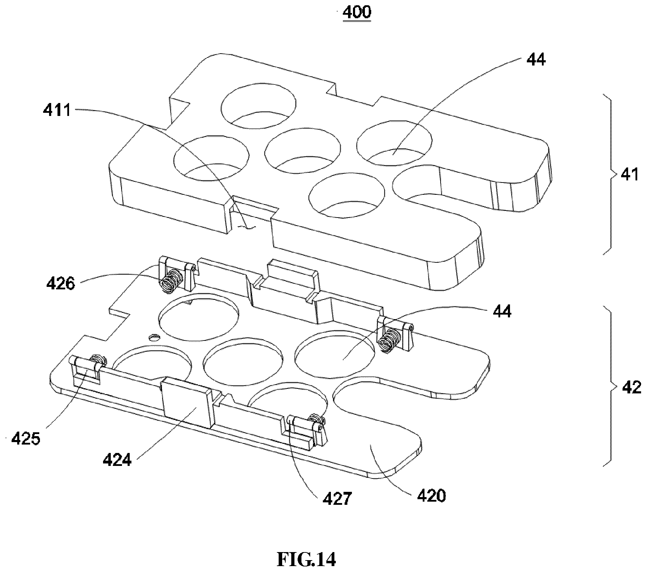

FIG. 13 and FIG. 14 illustrate a fifth embodiment provided by the present disclosure - a sterile isolation plate assembly 400. The sterile isolation plate assembly 400 includes a second clamping mechanism 42 and a sterile isolation plate 41 provided on an upper side of the second clamping mechanism 42.

A difference between the second clamping mechanism 42 and the clamping mechanism 100 is that a second clamping member 425 of the second clamping mechanism 42 is in a rotatable shaft/hole connection, vis its upper end portion, to a corresponding second clamping seat 427. Meanwhile, a second base plate 420 of the second clamping mechanism 42 has no elastic base.

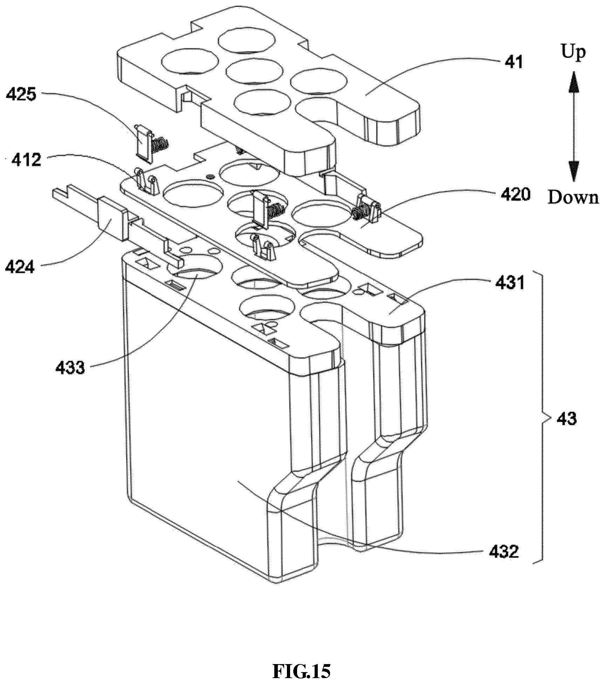

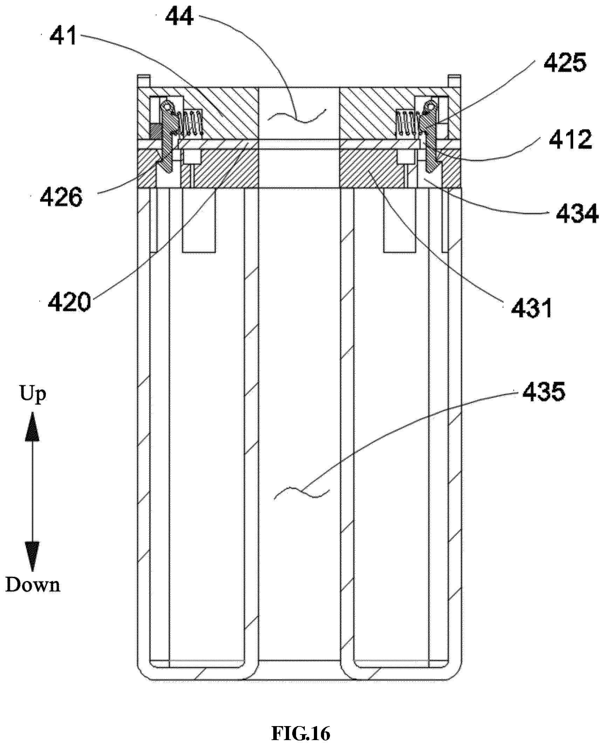

In combination with FIG. 15 and FIG. 16, the sterile isolation plate 41 is provided with a pair of pressing slots 411 that is respectively adapted to the pair of second pressing portions (not shown in the figures). The pair of pressing slots 411 allow the corresponding second pressing portions to be inserted thereinto and meanwhile allow a second force-bearing wall 424 to be exposed. The second force-bearing wall 424 is so configured that an outer surface thereof is flushed with a corresponding outer surface of the sterile isolation plate 41. A bottom surface of the sterile isolation plate 41 partially extends downwardly and forms an inner wall (not shown in the figures). This inner wall abuts against an end portion of each second elastic member 426 that is away from the corresponding second clamping member 425, so that the second elastic member 426 can apply a force to urge the corresponding second clamping member 425 into the second engaging/clamping position. In this example, the sterile isolation plate 41 is fixedly connected to the second base plate 420, and is provided with several second through slots 412 for engagement/clamping of an external clamping mechanism. In other embodiments, the sterile isolation plate 41 may also be provided with several through slots, so that another device with a clamping mechanism (such as the instrument cassette 300 described above) can be engaged/clamped with the sterile isolation plate 41 via its own clamping mechanism.

FIG. 15 and FIG. 16 show an application scenario of a sterile isolation plate assembly 400. The sterile isolation plate assembly 400 is engaged/clamped with the power box 43 and is located on an upper side of the power box 43. The power box 43 includes a top cover 431 provided at an upper portion, a power box housing 432 provided at a lower portion, and a power chamber 435 defined by the top cover 431 and the power box housing 432. The top cover 431 is fixedly connected to the power box housing 432, and is provided with several power-box clamping slots 434 for engaging/clamping with the second clamping mechanism 42. The power chamber 435 may accommodate a corresponding power device (including a battery, a motor, a transmission mechanism, and the like, which are not shown in the figures). The sterile isolation plate assembly 400 is provided with second power holes 44 penetrating the sterile isolation plate assembly 400. The top cover 431 of the power box 43 is provided with third power holes 433 corresponding to the second power holes 44. In practical application, a transmission device adapted to the second power hole 44 can be arranged within the sterile isolation plate assembly 400. When the sterile isolation plate assembly 400 is engaged/clamped with the power box 43, the power device in the power box 43 outputs power externally via the third power holes 443, the second power holes 44, and corresponding transmission devices. In other application scenarios, the sterile isolation plate assembly 400 may also be engaged/clamped with an instrument cassette without a clamping mechanism. Such instrument cassette includes a mounting base with several clamping slots. When the sterile isolation plate assembly 400 is engaged/clamped with such an instrument cassette, the sterile isolation plate 41 is located on a lower side of the instrument cassette, and the second clamping mechanism 42 is engaged/clamped with a mounting base of the instrument cassette.

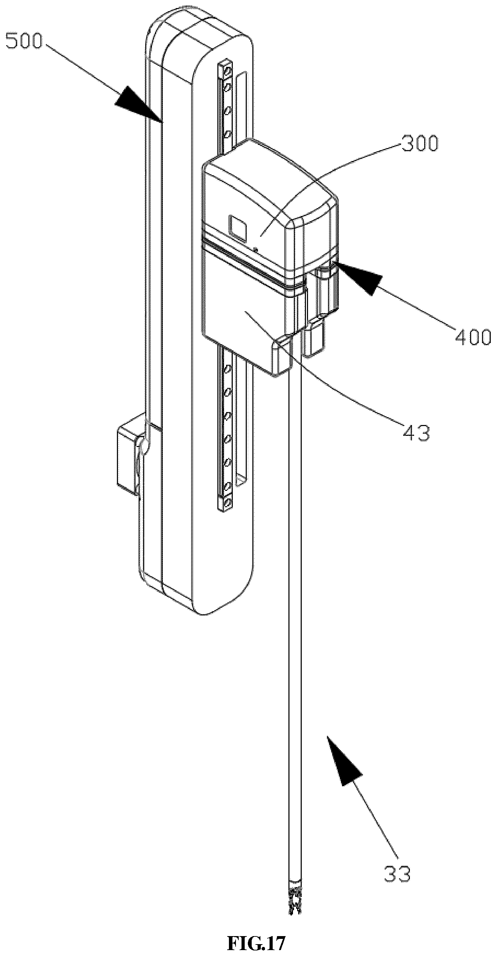

FIG. 17 show an application scenario of instrument cassette 300 and a sterile isolation plate 400. The sterile isolation plate assembly 400 is engaged/clamped with the power box 43 and is fixed on a corresponding mechanical arm 500. The mechanical arm 500 can drive the sterile isolation plate assembly 400 and the power box 43 to move up and down synchronously. The instrument cassette 300 is engaged/clamped with the sterile isolation plate 41 on the sterile isolation plate assembly 400 via the first clamping mechanism 32 (in this example, the sterile isolation plate 41 is provided with several clamping slots). Four first clamping members 325 provided by the first clamping mechanism 32 can provide a firm engagement/clamping with the sterile isolation plate 41, so as to avoid shaking or even detachment of the instrument cassette 300 from the sterile isolation plate 41. Similarly, four second clamping members 425 of the sterile isolation plate assembly 400 can provide a firm engagement/clamping with the top cover 431 of the power box 43, so as to avoid shaking or even detachment of the power box 43 from the sterile isolation plate 41. A power device in the power box 43 may supply power to the instrument transmission device in the instrument chamber 312 via the third power holes 433, the second power holes 44, the first power hole 3201, and the necessary transmission device. The instrument transmission device controls the effector 33 to operate by using this power. In other embodiments, four or more clamping members may be provided by the first clamping mechanism. It may be understood that if more clamping members are provided by the first clamping mechanism, the first clamping mechanism may be more firmly engaged/clamped with the corresponding sterile isolation plate.

In practical surgical operations, different instrument cassettes 300 equipped with different surgical tools are needed to be frequently replaced to meet requirements of the surgery. These instrument cassettes 300 are connected to other surgical instruments (such as the mechanic arm 500 and the power box 43 in this figure) through the sterile isolation plate 41 to prevent bacterial contamination between the two, thereby ensuring that the surgery may be carried out in a sterile environment.

The foregoing embodiments are only intended to illustrate the technical concept and features of the present disclosure, with an objective of enabling a person skilled in the art to understand the content of the present disclosure and implement the content accordingly. The protection scope of the present disclosure cannot be limited thereto. Any equivalent changes or modifications made in accordance with the spirit of the present disclosure shall be covered within the protection scope of the present disclosure.

## Claims

1. A clamping mechanism, comprising a base plate and a pair of clamping assemblies that are disposed opposite to each other on left and right sides, wherein
both clamping assemblies are fixedly connected to a same surface of the base plate;
each clamping assembly comprises a moving member, at least two clamping members, and several elastic members in one-to-one correspondence with the clamping members;
wherein each clamping member has an engaging/clamping position and a disengaging/unlocking position; the clamping member is configured to be able to clamp a third unit when in the engaging/clamping position and allow the third unit to detach when in the disengaging/unlocking position;
wherein each clamping assembly is so configured that: the moving member can move along the surface of the base plate under an action of an external force, drive the at least two clamping members to move from the engaging/clamping position to the disengaging/unlocking position, and compress the several elastic members to enable the at least two clamping members to move from the disengaging/unlocking position to the engaging/clamping position under an action of an elastic restoring force.

2. The clamping mechanism according to claim 1, wherein each moving member extends in a front-rear direction; the base plate is provided with two sets of guide rails that are slidably coupled with a pair of the moving members, respectively; the two sets of guide rails are both configured to extend along a left-right direction; and the moving member can move back and forth along the corresponding set of guide rails.

3. The clamping mechanism according to claim 2, wherein the moving member comprises a pressing portion extending along an up-down direction and a guiding portion extending along the left-right direction; and each guiding portion is slidably connected to the corresponding set of guide rails.

4. The clamping mechanism according to claim 1, wherein several clamping seats in one-to-one correspondence with the clamping members are fixedly disposed on the base plate; and each clamping member is provided with a rotating shaft to form a shaft/hole connection with the corresponding clamping seat via the rotating shaft.

5. The clamping mechanism according to claim 4, wherein
each clamping member comprises an upper end portion and a lower end portion that are respectively formed on two sides of the rotating shaft; each upper end portion is fixedly connected to or abuts against a corresponding moving member; each lower end portion is configured to have a hook-shaped structure; and
the moving member, when being actuated under an action of an external force, drives the upper end portion to rotate, so that the lower end portion of the clamping member may switch between the disengaging/unlocking position and the engaging/clamping position.

6. The clamping mechanism according to claim 5, wherein the base plate is provided with several through slots in one-to-one correspondence to the clamping members; each clamping member passes through the corresponding through slot and exposes the lower end portion; and the elastic member abuts against the upper end portion of the corresponding clamping member.

7. The clamping mechanism according to claim 6, wherein the elastic member is a spring; several elastic bases, a number of which is the same with a number of clamping members, that respectively abut against the elastic members are also provided on the base plate; and each elastic member applies a force to the corresponding clamping member to hold the clamping member at the engaging/clamping position.

8. The clamping mechanism according to claim 6, wherein the elastic member is an elastic leaf with an inverted U-shaped structure; the elastic member and the corresponding clamping member are constructed as an integral component; several elastic bases in one-to-one correspondence with the elastic members are also fixedly provided on the base plate; and each elastic member abuts against a corresponding elastic base and applies a force to the corresponding clamping member to hold the clamping member at the engaging/clamping position.

9. The clamping mechanism according to claim 6, wherein the clamping member is provided with a hollow portion; the elastic member is a torsion spring and is arranged in the corresponding hollow portion; each elastic member abuts against the corresponding clamping member and the base plate at its two end portions, respectively; and each elastic member applies a force to the corresponding clamping member to hold the clamping member at the engaging/clamping position.

10. An instrument cassette, comprising:
a mounting base;
a pair of first clamping assemblies that are arranged opposite to each other on left and right sides on the mounting base, wherein each first clamping assembly comprises a first moving member extending along a front-rear direction, at least two first clamping members that abut against or are fixedly connected to the first moving members, and several first elastic members whose number is the same with a number of the first clamping members; the first clamping member is elastically coupled with the mounting base via corresponding first elastic member, and has a first engaging/clamping position and a first disengaging/unlocking position; and each first moving member is provided with a first pressing portion to which an external force can be applied;
an instrument upper housing, wherein the instrument upper housing is of a box-shaped structure with a lower opening, and is provided with a pair of pressing slots that exposes a pair of the pressing portions, respectively; and
an instrument transmission device, provided on the mounting base and arranged within of the instrument upper housing, wherein
each first moving member is configured to be able to drive, under an action of an external force, corresponding first clamping member to move from the first engaging/clamping position to the first disengaging/unlocking position, and compress corresponding first elastic member to enable the first clamping member to move from the first disengaging/unlocking position to the first engaging/clamping position under an action of an elastic restoring force; and the first clamping member is configured to be able to clamp a third unit when in the first engaging/clamping position and allow the third unit to detach when in the first disengaging/unlocking position.

11. The instrument cassette according to claim 10, wherein each first clamping assembly is fixedly connected to an upper surface of the mounting base; the mounting base is provided with first through slots in one-to-one correspondence with the first clamping members; and each first clamping member passes through the corresponding first through slot and exposes its lower end portion.

12. A sterile isolation plate assembly, comprising:
a second base plate;
a pair of second clamping assemblies that are arranged opposite to each other on left and right sides on the second base plate, wherein each second clamping assembly comprises a second moving member extending along a front-rear direction, at least two second clamping members that abut against or are fixedly connected to the second moving members, and several second elastic members respectively abutting against the second clamping members with one-to-one correspondence; each second clamping member has a second engaging/clamping position and a second disengaging/unlocking position; and each second moving member is provided with a second pressing portion to which an external force can be applied; and
a sterile isolation plate fixedly connected to or detachably coupled to the second base plate, wherein the sterile isolation plate is provided with a pair of pressing slots that can allow a pair of the second pressing portions to be exposed, respectively; and a pair of second clamping assemblies are both located within the sterile isolation plate, wherein
the second moving member is configured to be able to drive, under an action of an external force, corresponding second clamping member to move from the second engaging/clamping position to the second disengaging/unlocking position, and enable the second clamping member to move from the second disengaging/unlocking position to the second engaging/clamping position under an action of an elastic restoring force; and the several second clamping members can clamp a third unit when in the second engaging/clamping position and allow the third unit to detach when in the second disengaging/unlocking position.

13. The sterile isolation plate assembly according to claim 12, wherein the second base plate is provided with several second through slots in one-to-one correspondence with the second clamping members; and the second clamping member passes through the corresponding second through slot and exposes its lower end portion.

14. The sterile isolation plate assembly according to claim 12, wherein the third unit is an instrument cassette; when the sterile isolation plate assembly is engaged/clamped with the instrument cassette, the sterile isolation plate is located on a lower side of the instrument cassette, and a pair of second clamping assemblies is engaged/clamped with a mounting base of the instrument cassette.

15. The sterile isolation plate assembly according to claim 12, wherein the third unit is a power box; when the sterile isolation plate assembly is engaged/clamped with the power box, the sterile isolation plate is located on an upper side of the power box, and the second clamping assembly is engaged/clamped with a top cover of the power box.
